Europäisches Patentamt

(19) European Patent Office

Office européen des brevets

(11) Publication number: **0 118 311**

**A2**

(12) **EUROPEAN PATENT APPLICATION**

(21) Application number: **84301459.8**

(22) Date of filing: **06.03.84**

(51) Int. Cl.³: **C 12 P 21/00**
**C 12 N 15/00, C 12 N 5/00**
**A 61 K 37/64**

(30) Priority: **07.03.83 GB 8306263**
**02.09.83 GB 8323639**

(43) Date of publication of application:
**12.09.84 Bulletin 84/37**

(84) Designated Contracting States:
**AT BE CH DE FR GB IT LI LU NL SE**

(71) Applicant: **Johnson, Sheena Margaret**
**7 Pebworth Road**
**Harrow Middlesex, HA1 3NB(GB)**

(72) Inventor: **Johnson, Sheena Margaret**
**7 Pebworth Road**
**Harrow Middlesex, HA1 3NB(GB)**

(74) Representative: **Silveston, Judith et al,**
**ABEL & IMRAY Northumberland House 303-306 High**
**Holborn**
**London, WC1V 7LH(GB)**

(54) Monoclonal factor having 5'-nucleotidase inhibitory activity.

(57) A monoclonal factor that has an inhibitory effect on lymphocyte plasma membrane 5'-nucleotidase, has a negative effect on immunoglobulin production, and has a molecular weight of 44,000 daltons ± 7,000, as determined by gel filtration and/or polyacrylamide gel electrophoresis; cell lines, especially continuous hybrid and transformed β-lymphocyte cell lines that are capable of producing the factor; and pharmaceutical preparations comprising the factor.

EP 0 118 311 A2

Croydon Printing Company Ltd.

## Monoclonal Factor Having 5'-Nucleotidase
## Inhibitory Activity

The present invention relates to a monoclonal factor directed against specific lymphocyte enzymes, to a process for its production, and to pharmaceutical preparations comprising the factor.

The present invention provides a monoclonal factor that has an inhibitory effect on lymphocyte plasma membrane 5'-nucleotidase, has a negative effect on immunoglobulin production, and has a molecular weight of 44,000 daltons $\pm$ 7,000, as determined by gel filtration and/or polyacrylamide gel electrophoresis.

The present invention also provides a monoclonal lymphocyte cell line that is capable of producing a factor of the invention. The cell line consists, for example, of lymphocytes that have been hybridised with a cell line suitable for hybridisation, for example, hybrid lymphocyte/myeloma cells, hybrid lymphocyte/plasma cytoma cells, and hybrid lymphocyte/hybridoma cells, or transformed lymphocyte cells.

The present invention further provides a method of producing a hybrid or transformed cell line of the invention, which comprises

(a) (i) fusing mammalian lymphocytes with a cell line suitable for hybridisation, for example myeloma, plasma cytoma or hybridoma cells, or

(ii) transforming mammalian lymphocytes and then, after (i) or (ii),

(iii) cloning resulting hybridoma or transformed cells and selecting those clones that produce an inhibitor of lymphocyte plasma membrane 5'-nucleotidase and that have a negative effect on immunoglobulin production; or

(b) cloning lymphocytes and selecting those that produce an inhibitor of lymphocyte plasma membrane 5'-nucleotidase and that have a negative effect on immunoglobulin production.

The present invention further provides a method of producing a factor of the invention, which comprises

(a) culturing a lymphocyte cell line of the present invention in the ascitic cavity of a host animal, and isolating the factor from the resulting ascitic fluid; or

(b) culturing a lymphocyte cell line of the invention in cell culture, and isolating the factor from the resulting cell culture supernatant.

The lymphocyte cell line is especially a B-lymphocyte cell line.

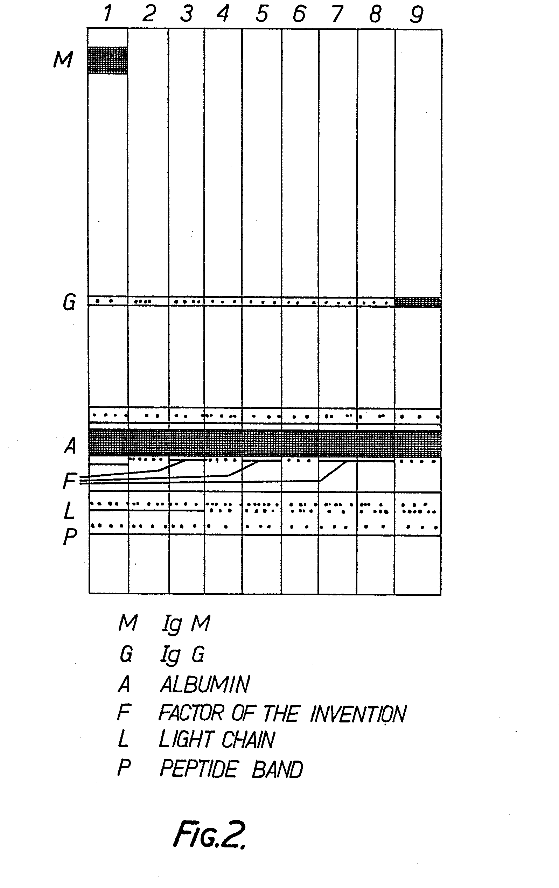

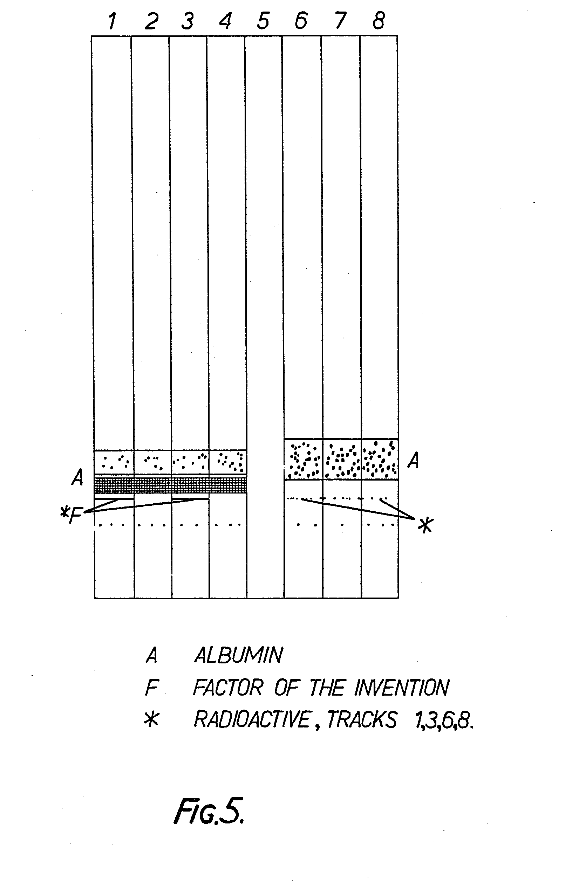

The molecular weight of the factor of the invention was initially found to be 60,000 daltons ± 5,000 by polyacrylamide gradient gel electrophoresis of the unreduced molecule, and this molecular weight was confirmed by autoradiography using the polyacrylamide gradient gel. The polyacrylamide gradient gel electrophoresis indicated that the factor of the invention is not an antibody, as there was no band for the factor at the position of the bands for IgG, IgE, IgA or IgM. (For further details, see the Example and Figures 2 and 5 of the accompanying drawings.) The factor does not appear to be any of the usual immunoglobulin fragments found in polyacrylamide gels, for example, light and heavy chains. It is possible that the factor of the invention could be a fragment of an immunoglobulin or a precursor for an immunoglobulin.

Further studies in which the factor is separated by gel filtration and by conventional polyacrylamide gel electrophoresis cf Laemmli, Nature 1970, 227, 680-685 now indicate that the factor of the invention has a molecular weight of 44,000 ± 7,000.

The factor of the invention is produced by certain lymphocytes. It is thought that the lymphocytes that are capable of producing the factor of the invention are most probably B-lymphocytes. Enriched or single-cell cultures of factor-producing cells may be prepared. These may be of use for hybridisation or transformation to produce factor-producing hybrid or transformed continuous cell lines suitable for large-scale cell culture. (The term "cell line" is used herein to mean continuous cell line.) Alternatively, the factor-producing cells for hybridisation may be obtained as a crude mixture, for example, of spleen cells.

If the cell line used for hybridisation does not produce immunoglobulin chains, then the production of such chains, for example, light chains, by a resulting hybridoma indicates a B-cell origin of the original factor-producing cell. When a resulting hybridoma does not produce immunoglobulin chains, it may be difficult to confirm that the hybridoma was derived from a B-lymphocyte. Other B-cell markers may be used to assist the classification of the original factor-producing cell.

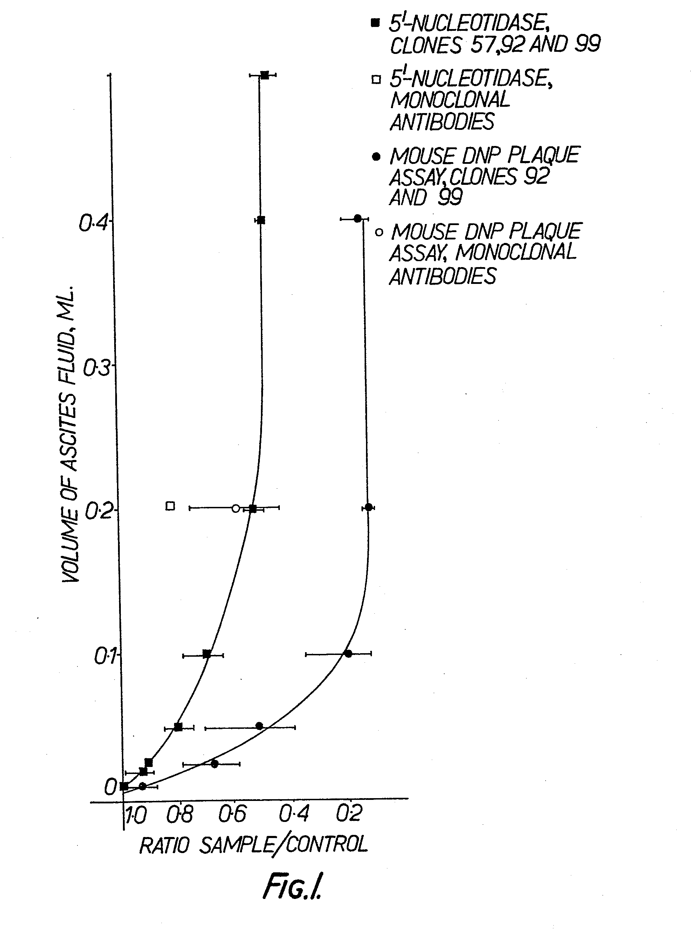

Lymphocyte plasma membrane 5'-nucleotidase is found in B-lymphocytes and in a sub-set of T-lymphocytes (see Rowe et al, (1979) Clin. Exp. Immunol. 36, 97-101). The factor of the invention has an inhibitory effect on this enzyme, but has no inhibitory effect on the 5'-nucleotidases found in the liver or on serum 5'-nucleotidase. The inhibitory effect on lymphocyte plasma membrane 5'-nucleotidase appears to have a maximum value in the region of 50%, for example, 50% $\pm$ 25%, for example, 50% $\pm$ 10%, for example 47% $\pm$ 4%, where $\pm$ 4% is the standard deviation from 5 determinations. The inhibitory effect appears to be non-competitive with 5'-AMP. (For further details see the Example and Figure 1 of the

- 5 -

0118311

accompanying drawings.)

The enzyme inhibitory effect occurs both inter-species and intra-species ie a factor of the invention obtained from one species of animal will inhibit lymphocyte 5'-nucleotidase from the same individual, from another individual of the same species, or from an individual of a different species. The inhibition of immunoglobulin synthesis is more species specific.

The negative effect of the factor of the invention on immunoglobulin synthesis is unexpected, particularly as a competitive inhibitor of lymphocyte plasma membrane 5'-nucleotidase ($\alpha,\beta$-methylene adenosine diphosphate) does not have any effect on immunoglobulin synthesis in vitro, see Rowe, M and Johnson, S.M. (1979) Biochem. Soc. Trans. 7, 997-998. The negative effect does not appear to be due to a toxic effect. The inhibitory effect on immunoglobulin synthesis is described in further detail in the Example.

It has also been found that the production of the factor of the invention appears to be stimulated, in some cases, by the presence of antibodies, and it is thought that the cells that are capable of producing a factor of the invention are lymphoctes involved in an antibody feedback syndrome.

In the process of the invention for the production of a cell line capable of producing the factor of the invention, mammalian lymphocytes may be fused with a cell line suitable for hybridisation. Suitable cell lines are well known, and include myeloma, plasma cytoma and hybridoma cells, for example.

The mammalian lymphocytes, including B-lymphocytes for example, spleen cells, may be obtained from an animal without any pre-treatment of the animal, but it may be advantageous to immunise the animal before the B-lymphocyte cells are obtained. As is known to those skilled in the art such immunisation may be carried out with any antigen. It may be advantageous to use as the antigen a cell line that is immunologically incompatible with the host animal, the cell line being capable of producing antibodies.

Examples of antibody-producing cell lines are antibody-producing myeloma cell lines, for example, human myeloma cell line U 266 B1 (Nilsson, K., Bennich, H., Johansson, G.S.O. and Ponten, J (1970) Clin. Exp. Immunol. 7, 477-489). Other suitable cell lines are known to those skilled in the art.

Alternatively, it may be advantageous to obtain the B-lymphocytes from an animal that has been immunologically primed with an immunologically compatible antibody or with a cell line that is immunologically compatible and is capable of producing immunologically compatible antibodies, or to prime B-lymphocytes in vitro with an immunologically compatible antibody.

Any myeloma (plasmacytoma) or hybridoma cells may be used for fusion with the lymphocyte cells, for example SP2/0 Ag 14 cells, P3-X 63 Ag 8 cells, or P3-NS 1-1 cells. These three cells lines are available from Flow Laboratories. Suitable host animals for cell lines are known to those skilled in the art. All three of the cell lines mentioned above are compatible with the commercially available Balb/c mice.

(The term "known" used herein means in actual use in the art or described in the literature of the art.)

Methods for fusing lymphocytes and myeloma cells and other suitable cell lines are well known, see for example, Kohler et al, Nature (1975) 256, 495-497; Kohler et al, Eur. J. Immunol. (1976) 6, 511-519 and Hochkeppel et al, Eur. J. Biochem. (1981) 118, 437-442. Any of these methods may be used in the process of the invention.

Instead of fusing lymphocyte and myeloma cells, mammalian B-lymphocytes may be transformed, for example with the Epstein Barr virus. Transformation processes are known in the art, see for example, Crawford et al, The Lancet (i) 1983,386 - 388.

Methods of cloning hybrid cells resulting from fusion, or transformed cells are also well known, see for example, Kohler et al (Nature loc cit); Hochkeppel et al (loc cit) and Secher et al, Nature (1980) 285, 446-450.     It has been found to be advantageous to use the method of Kohler et al (Nature loc cit) with the following modification; after the second sub-clone, the hybrid or transformed cells are injected into the ascitic cavity of another animal immunologically compatible with the host animal.  The resulting ascitic fluid contains the factor of the invention.  The spleen of the injected animal may be removed and cultured aseptically, and then the hybrid or transformed cells may be recloned in culture.  The resulting cell cultures have improved stability.  The passage through a mammalian host during cloning may also be carried out at a different stage of the cloning procedure.

As the production of the factor of the invention appears to be stimulated by antibodies, it is advantageous to have additional antibody present during the cloning procedure, since positive clones may be selected more easily.

In the fusion, transforming and cloning procedures, the hybrid or transformed cells are selected by their ability to inhibit lymphocyte plasma membrane 5'-nucleotidase activity or by their negative effect on immunoglobulin synthesis, or by both of those effects.  Methods of determining both of the above effects are known, and any method may be used for selecting suitable cells. A method for determining lymphocyte plasma membrane 5'-nucleotidase is described by Avruch et al (1971) Biochim. Biophys. Acta $\underline{233}$, 334-3-7. A modification of this assay is described in the Example herein. Inhibition of immunoglobulin synthesis may be carried out in vivo, but it is generally more convenient to use an in vitro determination, examples of which are well known, for example, the plaque assay described in Hudson and Hay, "Practical Immunology", 2nd. Edition, Blackwell Scientific Publications Ltd. 1980. It is advantageous to produce a factor of the invention from a cell culture rather than from the ascitic fluid of a host animal. Accordingly, the invention provides a method of producing a factor of the invention which comprises cultivating a clone of hybrid or transformed cells that is capable of producing a factor of the invention in a suitable culture medium, and isolating the resulting factor from the supernatant of the culture. As indicated above, the production of a factor of the invention appears to be stimulated by the presence of antibodies, so it is very advantageous to include an antibody in the culture medium.

Suitable culture media for continous cell lines of the present invention are, for example, RPMI 1640, Dulbecco's Minimum Essential Medium, and Enriched Dulbecco's Minimum Essential Medium, forming the basis of Iscove's medium, with the addition of 10%
inactivated foetal calf serum.  Inactivation of the
calf serum was carried out by heating the serum at
$70^{\circ}C$ for 10 minutes, which also destroyed the bovine
5'-nucleotidase activity.  Culture of the cell lines is
carried out in known manner.  (Cell culture techniques
are well known, see, for example, Kohler et al, loc
cit, and Hochkeppel, loc cit.)

If desired, the factor of the invention, which may
be obtained from the supernatant of a culture of hybrid
or transformed cells of the invention, may be purified.
Methods suitable for purifying molecules of the size of
the factor of the invention are known.  Affinity
methods may be particularly useful.  B-lymphocytes have
been found to adsorb the factor of the invention, and
this may be used in an affinity method.

Using the process of the invention, Balb/c mice
were immunised with human myeloma U 266 B1 living
cells.  The resulting spleen B-lymphocytes were fused
with SP2/O Ag 14 cells.  The resulting hybrid cells
were selected and cloned and the cloned cells were
selected on the basis of their ability to inhibit
lymphocyte plasma membrane 5'-nucleotidase.

There were obtained three cell lines that produced
a factor capable of inhibiting lymphocyte
5'-nucleotidase (clones 57, 92 and 99).  Of these,
clone 57 produces a kappa light chain in addition to the factor of the invention. This proves the B-cell origin of the fusing lymphocyte. On present evidence, it is considered highly probable that clones 92 and 99 are also derived from B-lymphocytes. Cell lines 57, 92 and 99 were each grown for 20 days with 5 changes of an antibiotic-free medium. All three cell lines grew well and showed no evidence of bacterial infection, or of extra-nuclear DNA when stained with a suitable dye, for example, HOE 33258, indicating the absence of mycoplasma infection.

The production of a factor of the invention by cell lines 99 and 57 is enhanced markedly by the presence in the culture medium of a diluted (x5) supernatant from a monoclonal mouse IgM hybridoma, and clone 99 is also stimulated by diluted (x5) supernatant from a monoclonal $IgG_1$ mouse hybridoma. In both cases the monoclonal mouse antibodies were directed against Trypanosoma cruzi antigens.

It is surprising to find that B-lymphocytes produce an apparently non-antibody switching factor, as normally it seems that B-lymphocytes produce antibodies, and that the T-lymphocytes produce switching factors. It will be appreciated that, in analogy to monoclonal cell lines and the resulting antibodies, each monoclonal cell line of the present invention may produce a factor that is capable of inhibiting lymphocyte plasma membrane 5'-nucleotidase

- 12 -    0118311

and which has a negative effect on immunoglobulin production, but the individual factors may differ slightly in their structure, as do antibodies. They may also differ qualitatively and quantitatively in their effects. The structure and degree of effect may also vary from species to species, the present invention providing all mammalian factors, including human factors. The present invention accordingly includes all members of such a "family" of factors.

The negative effect of the factor of the invention on immunoglobulin production has considerable practical importance. It appears that the factor of the invention may be able to alter temporarily the ability of the immune system to react to antigens. The negative effect, which appears to be a modification of or an imitation of a natural process, can be used for example, in transplantation procedures, in the treatment of autoimmune diseases, including various allergic conditions, and in the treatment of those instances where cancer is enhanced by the activity of the immune system, and in any condition in which suppression of any part of the humoral immune response is desirable. Alternatively the factor of the invention may exert an inhibitory or indirectly cytotoxic effect on the cancer cells themselves.

The present invention accordingly provides a pharmaceutical preparation which comprises a factor of

- 13 -                    0118311

the invention in admixture or conjunction with a pharmaceutically suitable carrier.  The preparation may be in any pharmaceutical form, for enteral or parenteral administration, for example, for oral, rectal, intranasal, intravenous or intraperitoneal administration or for administration by inhalation.

Examples of preparation forms are tablets and capsules, suppositories and pessaries, inhalable sprays and powders, and preparations for injection or infusion, including preparations for reconstitutions before use.

Preparation may be in unit dosage form.

The invention also provides the therapeutic use of a factor of the invention, for example, for the indications given above, for example, for the treatment of humans.

The following Example illustrates the invention.

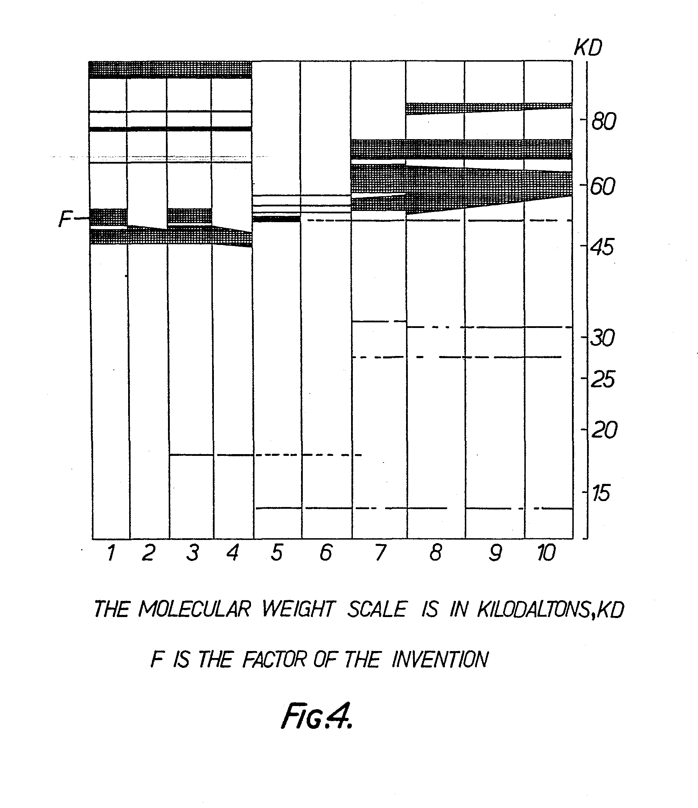

Reference in drawn in the Example to Figures 1 to 5 of the accompanying drawings.

EXAMPLE:

Materials

Adenosine 5'-monophosphate, levamisole and sodium ß-glycerophosphate were obtained from Sigma, and $^3$H-adenosine monophosphate and $^{35}$S methionine from the Radiochemical Centre, Amersham. All other chemicals used were reagent grade. The mice were pathogen free. The cell culture medium, RPMI 1640 and Iscove's complete medium were obtained from Flow Laboratories together with L-glutamine and the antibiotics penicillin, streptomycin and gentomycin. The foetal calf serum, DMEM and Iscove's enriched DMEM came from Gibco. Lymphoprep came from Nyegaard and Co., Norway. The Gradipore continuous concave gradient gels were precast, and were brought from Universal Scientific Limited.

Methods

Unless otherwise stated, the cell culture medium used were RPMI 1640, buffered with bicarbonate and supplemented with 2mM L-glutamine and 10% heat inactivated foetal calf serum. 100 iu penicillin, 100µg/ml streptomycin and 0.16 mg/ml gentomycin were added, and the cells grown at $37^\circ$C in an incubator with air enriched with 5%$CO_2$.

Whole living cells from the human myeloma cell line U266 B1 (Nilsson, K., Bennich, H., Johansson, G.S.O. and Ponten J. (1970) Clin. Exp.

Immunol. 7, 477-489) were used to immunise female Balb/c mice about six weeks old. $10^6$ cells were injected intraperitoneally, at weekly intervals for 3 weeks. Seven days later the grossly enlarged spleens were removed. $10^8$ spleen cells were fused with $10^7$ SP2/O-Ag 14 cells (Schulman, M., Wilde, C.D. and Kohler, G. (1978) Nature 276, 269-270) using polyethylene glycol, and the resulting hybridomas positively selected for the presence of the enzyme hypoxanthine guanine phosphoribosyl transferase as described by Kohler and Milstein, (1975) Nature 256, 495-497. The technique is well recognised, further details are given in "Immunochemistry in Practice", A. Johnstone and P. Thorpe, Blackwell Scientific Publications Ltd, 1982).

The hybridomas were grown in 2 ml Costar plates over Balb/c peritoneal macrophages. The supernatants were tested biochemically for the presence of an inhibitor to human lymphocyte 5'-nucleotidase.

5'-Nucleotidase assay Cells were counted electronically using a Coulter counter. $4 \times 10^4$ U266 B1 cells or $3 \times 10^5$ human tonsil lymphocytes were incubated with 1 ml supernatant in disposable plastic tubes for at least one hour in melting ice. 12 ml ice cold 0.145M saline buffered with 10 mM tris chloride, pH 7.1 ($20^{\circ}$C) was added and the cells centrifuged at 200g for 15 minutes in a refridgerated centrifuge. The supernatant was removed by suction, and the 5'-nucleotidase activity of the cell pellet was assayed by a method adapted from Avruch and Wallach (Biochim. Biophys. Acta 233, 1971, 334-347).   The assay mixture contained 0.02 mM adenosine monophosphate with 0.2 μ Ci/ml tracer $^3$H – adenosine monophosphate, 1mM levanisole, 0.06 mM β-glycerophosphate, 10 mM magnesium chloride and 100 mM glycine sodium hydroxide buffer, pH 8.5.   The cells were suspended in 1 ml of the assay mixture and incubated at 37$^{\circ}$ in a water bath for 20 minutes.   Afterwards the tubes were cooled in melting ice for 5 minutes, and 0.3 ml 0.15 N zinc sulphate was added, followed by 1 ml 0.15 N barium hydroxide solution to precipitate the unreacted adenosine monophosphate.   The mixture was centrifuged at 300 g for 10 minutes.   1 ml of the supernatant was withdrawn, and counted for $^3$H using 10 ml Unisolve in a Packard scintillation counter.   The 5'-nucleotidase activity of the cells in n moles/hr/10$^6$ cells was calculated from the relation:-

$$5' \; N = \frac{(X - B) \times 6}{(S - B) \times N} \times 10^5$$

Where X, B and S were the count rates of the sample, blank and standard respectively, and N the number of cells present in the sample. The fractional inhibition was calculated from the ratio of the 5'-nucleotidase

activity on the cells after treatment with the supernatant compared to duplicate samples of the cells treated with the culture medium only. It is expressed as the ratio sample/control, the lower the fraction the greater the inhibition. It was previously expressed as (1-Sample/control).

Results

Initially eight positive clones were obtained from 109 wells containing hybridomas. Of these three were selected by clonal competition. These hybridomas, numbers 57, 92 and 99, were grown in 75 cm$^2$ polystyrene tissue culture flasks, and the adherent cells were retained each time the medium was changed. The supernatants remained able to inhibit the tonsil 5'-nucleotidase activity for several months in culture without cloning. The use of U 266 B1 as a test cell was discontinued when it was found to be infected with mycoplasma. The hybridomas were cloned twice by the method of limiting dilution, using mouse peritoneal macrophages or spleen cells as a feeder layer. Satisfactory results were obtained at an initial concentration of 2 hybridoma cells per well of a 96 well Costar plate. The cloning method is described in "Immunochemistry in Practice", A. Johnstone & R. Thorpe, Blackwell Scientific Publications, 1982. The hybridoma cells were then injected into the peritoneal cavity of pristane primed Balb/c mice, using about

$10^6$ cells per mouse. The tumour was harvested 10-20 days later, and the cell suspension containing 10 units of heparin/ml was centrifuged at 200g for ten minutes and the cell supernatant stored frozen at $-20^oC$. If the supernatant was not to be used in experiments to measure antibody production, 0.02% sodium azide was added. The spleens of the mice were harvested aseptically, and a cell suspension prepared as in the fusion experiments. These were cultured, and the hybridoma cells were recovered from most spleens. These were recloned, and the supernatants tested. About 60-80% of the clones were positive, which was an improvement on earlier 10-15% positives. Antibody produced by the splenic lymphocytes may be the cause. Although the hybridomas grew well in RPMI, it was noted that the uncloned hybridoma 99 produced aproximately 20 times as much factor if grown in a mixture of 40% RPMI (including the 10% foetal calf serum) and 60% Iscove's complete medium. However, the other two hybridomas did not survive in this mixture. It is now thought that the factor-producing clone may have been stimulated by an antibody produced by another hybridoma in the cell mixture.

Inhibition of human tonsil lymphocyte 5'-nucleotidase by the factor of the invention. Enlarged human tonsils which had been removed from children were kept on ice, and a cell suspension in phosphate buffered saline was prepared from them about two hours later. The cells were washed once, then the lymphocytes were separated on a lymphoprep gradient according to the manufacturer's instructions. After harvesting, the cells were washed twice in phosphate buffered saline, and then frozen slowly at a concentration of $5x10^7$ cells/ml in the cell culture medium to which 10% dimethyl sulphoxide had been added as a cryopreservative. They were stored in liquid nitrogen until required; the enzyme activity of 5'-nucleotidase was found to be unaffected by this treatment.

The required volumes of the azide treated ascites fluid obtained as described previously containing the factor of the invention were measured into test tubes and diluted to 1 ml with cell culture medium or 1% bovine serum albumin in 10 mM tris buffered saline, pH 7.1 ($20^{\circ}$C) containing 0.02% sodium azide. The mixtures were kept on ice. Tonsil cells were unfrozen at $37^{\circ}$, and 10 µ L aliquots from the vial added to the ice cold experimental samples. They were allowed to stand on ice for at least one hour. The remaining tonsil cells were frozen again in the vapour from the liquid nitrogen. After incubation, the cells were then washed with cold tris saline as described in the assay for the hybridoma supernatants, and assayed for 5'-nucleotidase activity as described previously. All assays were done in duplicate. If less than 1 ml of cell culture medium or 1% bovine serum albumin was used, it was necessary to have some protein present in the washing mixture to prevent cell loss by adsorption to the plastic tube. Controls contained no ascites fluid. The results are shown in figure (1) of the accompanying drawings. No difference between the factor(s) produced by the three hybridomas was noticed. The fractional inhibition of the enzyme was not more than 0.47 ± 0.04, where 0.04 represents the standard deviation from five samples. The enzyme assay as described previously only gave results linearily proportional to the quantity of enzyme present if under 35% of the total substrate concentration was used up, but up to this limit the inhibition curve produced by the ascites fluid was not affected by the total number of tonsil cells in the assay, nor by using a lower volume of the protein solution. For example, 100 μ L of the ascites fluid containing the factor of the invention gave an enzyme inhibition of 0.74 ± 0.05 (7) when diluted 10:1, and an inhibition of 0.72 ± 0.06 (4) when diluted 1:1. This result shows that the binding constant between the enzyme and the factor of the invention is quite high.

The factor of the invention is a non competitive inhibitor of 5'-nucleotidase activity using 5'-adenosine monophosphate as a substrate. The apparent Michaelis - Menten constant of the tonsil

0118311

lymphocyte 5'-nucleotidase was measured by biochemical techniques. The enzyme assay was performed at different concentrations of adenosine monophosphate, keeping the concentrations of the other substances in the assay medium constant. In one case, equal members of tonsil cells were incubated for one hour in 1 ml 1% bovine serum albumin at $0^{o}$C with and without 50 $\mu$ L ascitic fluid from clone 99. Other concentrations of ascitic fluid from the three clones were also used. After the usual wash, equal numbers of cells were added to the assay medium containing the different dilutions of adenosine monophosphate. The Michaelis-Menten constants $K_m$ and $V_{max}$ were calculated from data by non-linear regresssion analysis see, for example, Cornish-Bowden and Eisenthal, Biochem J.(1974) 139, 721-730. $V_{max}$ was lowered significantly by the presence of the factor, but there was no significant difference in the apparent $K_m$, which was 9.6n moles of adenosine monophosphate per hour per $10^{6}$ cells.

Other cell determinations.

Human circulating lymphocytes were purified from other blood cells by centrifuging over lymphoprep as described for the tonsil cells. T cells were prepared from the purified circulating cells by removing "sticky" B-cells and monocytes by passage over a nylon wool column. They were found to be 97% pure when studied in a fluorescence activated cell sorter using the monoclonal antibody T cell marker OKT 3. Human liver cells were obtained as a washed but otherwise unpurified suspension from a cadaver who had been dead for 16 hours. The cells did not appear viable, but the erythrocytes were unlysed, and considerable 5'-nucleotidase activity remained. The cell suspension was stored frozen, as described for the tonsil lymphocytes. Chronic lymphocytic leukaemia cells were prepared from the patient's blood as were the normal circulating lymphocytes. They were unusual in that they had a supranormal 5'-nucleotidase activity, (Quagliata, F., Faig, D., Conklyn, M, and Silber, R.(1974) Cancer Res. 34, 3197-3202). Mouse spleen cells were washed but not further purified, as were mouse bone marrow cells extracted from mouse femurs.

The 5'-nucleotidase activity of the tonsil cells was still inhibited by the factor of the invention if 100 μ L of human plasma was present during the incubation. This contained a nucleotidase activity of 22.8 n mole/hr.

No difference in specificity between the factor(s) produced by the three clones has yet been found as regards lymphocyte 5'-nucleotidase activity.

The inhibition of the 5'-nucleotidase activity on mouse lymphoid cells would not be expected if the factor of the invention was a monoclonal antibody produced in the same strain of mouse.

Table showing the inhibition of 5'-nucleotidase
by the factor(s) of the invention

| Human Cells | 5'N | 57 | 92 | 99 | Volume Ascitic Fluid,ml |
|---|---|---|---|---|---|
| Tonsil cells | – | 0.68+0.10 | 0.70+0.08 | 0.73+0.06 | 0.1 |
| Chronic lympho-cytic leukaemia | 17.0 | 0.73 | 0.78 | 0.83 | 0.1 |
| T cells | 22.0 | 0.60 | 0.57 | – | 0.25 |
| circulating | 4.2 | 0.62 | 0.62 | 0.55 | 0.1 |
| Circulating lymphocytes | 8.5 | – | – | 0.53 | 0.2 |
| (mononuclear cells) | 5.4 | 0.62 | 0.63 | 0.67 | 0.1 |
| Tonsil + 0.1ml serum | 22.8 | 0.70 | 0.75 | 0.79 | 0.1 |
| Liver | 5.5 | 0.99 | 1.00 | 0.97 | 0.1 |
| " | 7.8 | – | – | 0.91 | 0.2 |
| Cat lymph node | 6.6 | 0.60 | 0.64 | 0.63 | 0.1 |
| Mouse Spleen | 1.28 | (0.48) | (0.69) | 0.58 | 0.2 |
| Mouse Bone Marrow | 1.6 | (0.47) | (0.83) | 0.63 | 0.2 |
| Rabbit Spleen | 12.6 | 0.81 | 0.77 | 0.81 | 0.1 |
| Rat Spleen | 7.5 | 0.75 | 0.76 | 0.74 | 0.1 |

The results in brackets were not performed in
duplicate. The 5'-nucleotidase activity is expressed in
n moles substrate utilized per hour, and the activity of
the enzyme is much lower generally in mouse as opposed
to human lymphoid cells. The results should be compared
to those for tonsil cells in Figure (1).

Polyacrylamide gel electrophoresis.

Precast continuous concave gradient gels were used.
The polyacrylamide gradient increased from 2.5 to 28%
over the 13cm length of the gel. These give very good resolution of complex protein mixtures, the larger the protein the sooner it is trapped by the decreasing pore size of the gel. Gels were run at 50 V for 24 hours at $16^{o}C$, in 0.1% sodium dodecyl sulphate, buffered at pH 8.3 with 0.1M tris buffered glycine. A 4.5% polyacrylamide gel was cast on top of the main gel, to contain the samples. This gel contained 0.1% sodium dodecyl sulphate, and was buffered at 6.8 with 0.12 M tris chloride. The samples were prepared in a buffer containing 5% glycerol, 0.0625M tris chloride, pH 6.8, 0.005% bromophenol blue 1% sodium dodecyl sulphate, and, if the proteins were to be reduced, 1% ß-mercapto-ethanol. Samples were boiled in this mixture for 1.5 minutes before being applied to the gel. Some samples were previously adsorbed with tonsil cells; in the first gel, figure (2) of the accompanying drawings, 0.05 ml of the ascites fluid containing the factor of the invention was adsorbed for one hour on ice with $2 \times 10^{7}$ tonsil lymphocytes which had been unthawed and washed once in 15 ml tris buffered saline, pH 7.1. The tonsil cells were centrifuged at 300 g for 10 minutes, and the adsorbed sample harvested, any dilution being noted. For the second gel, figure (5), 0.2 ml of the factor-containing sample was adsorbed with $10^{8}$ tonsil lymphocytes. Gels were stained after the electrophoresis with 0.025 g Coomassie Brilliant Blue in 45% methanol, 10% glacial acetic acid and 45% water for 24 hours then destained for 48 hours in 7.5% acetic acid and 5% methanol. The gel shown in figure (2) had the equivalent of 4 μ L ascites fluid applied to each track, making allowance for the sample dilution if necessary. Track 1 contains an IgM monoclonal antibody, and track 9 an Ig $G_1$ monoclonal antibody, both a gift from Dr. Brian Anderton. Track 2 contains adsorbed 57 and track 3 unadsorbed 57. Tracks 4 and 5 contain adsorbed and unadsorbed 92, track 6 adsorbed 99 and tracks 7 and 8 two different samples of unadsorbed 99. It will be seen that there is no evidence of a factor as heavy or heavier than $IgG_1$, 146,000 Daltons, which occurs in all three hybridomas and which is adsorbed by tonsil lymphocytes. However below albumin, molecular weight 69,000 Daltons, just such a factor does appear, although it is faint. It should however be recalled that this factor, unlike the monoclonal antibodies, will react with the lymphocytes of the tumour host mouse. Albumin in its unreduced form runs rather further in this gradient gel than would be expected, possibly due to a shape factor. The gel also shows some light chain immunoglobulin secreted by the Ig M monoclonal and hybridoma 57. Light chain was also seen in gradient gels of the culture medium supernatant of 57 and 92, but due to difficulties with albumin the factor of the invention was obscured. No other evidence for a light chain from 92 was obtained.

Light chain was not adsorbed by tonsils.

Autoradiograph Hybridomas 92 and 99 were grown to form monolayers in a 2 ml Costar plate, then each well was washed with minimum essential medium, and left for 24 hours in methionine-free medium containing the usual antibiotics, L-glutamine, 10% inactivated foetal calf serum and 50 µ Ci $^{35}$S-methionine. Each hybridoma gave 3 ml supernatant, to which was added 0.5 ml of the appropriate ascites fluid. The mixtures were dialysed against 500 ml 10 mM tris saline, pH 7.1, containing 0.02% sodium azide for 16 hours at 8$^o$C, then the cell supernatants were stored frozen. Samples containing 0.02 ml were run on a gradient gel as described previously. The gel was stained and photographed as described previously. The result is shown in figure (5) of the accompanying drawings. After soaking twice in 100 ml dimethyl sulphoxide for about 12 hours, the gel was left overnight in a 17% solution of the scintillant PPO in dimethyl sulphoxide. The gel was then transferred to water containing 0.2% glycerol. After the PPO had precipitated, the gel was dried onto filter paper, transferred to a cassette and exposed to double sided Fuji X-ray film for 24 hours at -76$^o$C. On development, the bands shown on the figure (5) could be seen, confirming the molecular weight and hybridoma origin of the factor of the invention. Track (1) and track (2) contained unadsorbed and adsorbed hybridoma 92 supernatant, and tracks (3) and (4) unadsorbed and adsorbed hybridoma 99 supernatant. Tracks (6) and (7) were unadsorbed and adsorbed reduced 92 hybridoma supernatant, and (8) unadsorbed and reduced 99 hybridoma supernatant.

Molecular weight determination.

(a) Gel filtration.

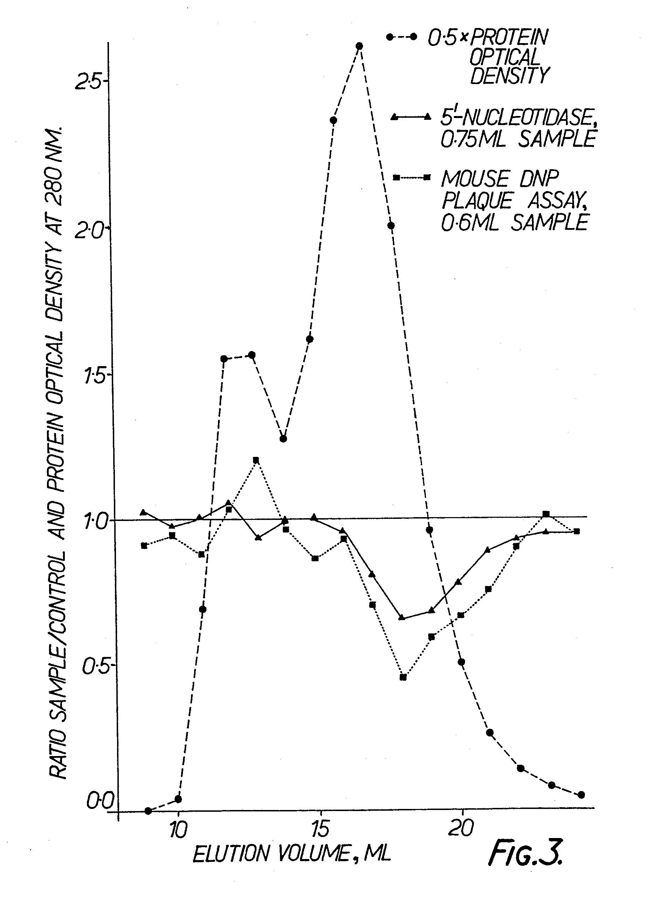

A Biogel P-150 column 47x0.79 cm was used at a flow rate of 3 ml/hr to fractionate 1 ml samples of ascitic fluid containing the factor of the invention. The eluting solvent was 10 mM tris saline, pH 7.1, containing 2 mM magnesium chloride. 1 ml samples were collected. The optical densities of these samples were measured at 280 nm, and their ability to inhibit the 5'-nucleotidase activity of human tonsil cells was assayed as described previously. Before assaying, 0.5 ml 1% bovine serum albumin in tris buffered saline was added to each tube. The column was calibrated by standard biochemical techniques using bovine serum albumin (66,200), ovalbumin (45,000), human carbonic anhydrase (28,500), myoglobin (17,200) and cytochrome C (12,300). The molecular weight of the factor was found from a plot of $K_d$ against log molecular weight, where $K_d$ = (Elution volume of protein - Void volume)/(Total volume of column - Void volume). The elution profile of the column and the ability of 0.75 ml samples to inhibit 5'-nucleotidase activity is shown in figure 3.

Polyacrylamide Gel electrophoresis.

The molecular weight of the factor was measured by sodium dodecyl sulphate polyacrylamide gel electrophoresis as described by Laemmli, Nature (1970) 227 680-685.    A 12.5% polyacrylamide gel was used, with the same protein molecular weight standards as the column.  A protein band approximately the same molecular weight as reduced ovalbumin appeared in column fractions showing a 5'-nucleotidase blocking activity, and this band corresponded to one of the strongest bands on an autoradiograph from the cell culture supernatant, obtained as described previously but run on a 12.5% gel with the Laemmli system.  The gels are shown in diagram 4, where tracks 1 and 2 show the supernatant from clone 92 not absorbed and absorbed by tonsil cells, tracks 3 and 4 show the same for clone 99 and tracks 5 and 6 are unabsorbed and absorbed clone 99 supernatants which have been reduced by mercaptoethanol.  Tracks 1-6 show the radioactive bands from the autoradiograph.  Track 7 is a reduced column fraction containing the factor, and 8,9 and 10 are successive unreduced column fractions containing the factor.  The molecular weight scale was calculated from a plot of log molecular weight against distance moved for the calibrating standard proteins.

The molecular weight of the factor appeared to be unaffected by the reduction with mercaptoethanol, which suggests that it is not a light chain dimer or Bence Jones protein.

Two determinations of the molecular weight by gel filtration gave a mean value of 42,000 $\pm$ 5,000 daltons, and two determinations by sodium dodecyl sulphate polyacrylamide gel electrophoresis gave a mean molecular weight of 46,000 $\pm$ 5,000 daltons.   The factor of the invention was therefore found to have a molecular weight of 44,000 $\pm$ 7,000 daltons.

Effect of the factor of the invention on antibody production.

The method used to measure antibody production was the Cunningham modification of the Jerne plaque assay, (A. J. Cunningham and A. Szenberg, Immunology 1968 14, 599-600).   Methods of labelling sheep red cells with dinitrophenol groups and protein A are described in Hudson and Hay, "Practical Immunology" 2nd edition, Blackwell Scientific Publications Ltd. 1980, which also describes the plaque assays.   During the labelling of the sheep red cells with picryl sulphonic acid, it was found advantageous to use an isotonic phosphate buffer containing 5.616 g/l $NaH_2PO_4.2H_2O$ and 16.188 g/l $Na_2HPO_4$, the pH was 7.2. The original method used a cacodylate buffer.

Mice were primed with dinitrophenylated keyhole limpit haemocyanin and pertussis vaccine as described by Hudson and Hay, loc.cit. After 7 days the spleen was removed, and a lymphocyte suspension prepared.   This was optimal for the primary IgG response. For IgM plaques 5 days immunisation was required, and for the secondary IgG response the spleens were removed ten days after a second immunisation at least 3 weeks after the first.

In an initial experiment a double blind trial was performed on azide free ascites fluid from hybridomas 92 and 99 at a dilution of 1:10 and 1:40.

The cells were incubated in complete RPMI 1640 with the diluted ascitic fluid for 1.5 hours at 37°. In this case only the sheep red cell targets had a dinitrophenylated Fab'fragment attached to them, Hudson and Hay, loc. cit. After washing in complete RPMI, the lymphocytes were incubated with 1/50 dilution of goat anti DNP IgG, guinea pig complement and the target red cells, and the plaques left to develop at 37°. The controls were not exposed to the diluted ascitic fluid. In the following table the inhibition is expressed as (Number of plaques in sample/Number of plaques in control). It was previously expressed as (1-(sample/ control) x 100

| Clone | Vol. Ascitic Fluid | Dilution | Inhibition |
|-------|--------------------|----------|------------|
| 92 | 0.1 ml | 1:10 | 0.42 |
| 92 | 0.025 ml | 1:40 | 0.33 |
| 99 | 0.1 ml | 1:10 | 0.41 |
| 99 | 0.025 ml | 1:40 | 0.13 |

It is not known why the smaller volume of ascites fluid produced the greater inhibition of immunoglobulin production.

Figure (1) shows the dose response curve for the ascitic fluids of 99 and 92 against the primary response of mouse lymphocytes to DNP. The target sheep red cells were labelled with picryl sulphonic acid as described previously. It did not matter whether the preliminary incubation with the factor was at $0^o$ or $37^o$, nor whether clone 99 or clone 92 was used. The results show the mean and standard deviation of at least 5 experiments. Ascitic fluid from 57 was also inhibitory. Live cell counts were performed on the lymphocytes after washing, using a Coulter Counter and Zapoglobin to remove the sheep red cells. The ascitic fluid was found not to be cytotoxic either for the mouse lymphocytes or for the tonsil lymphocytes used in the protein A experiments. Normally $10^6$ lymphocytes were used per sample. Both the incubation medium and the washing medium were Iscove's DMEM with 10% inactivated foetal calf serum. The assay was run in Iscove's DMEM.

Figure (3) shows the inhibition of the secondary immune response to DNP by mouse lymphocytes. In these experiments 0.5 ml of the sample eluate was incubated with 0.2 ml inactivated foetal calf serum and 0.5 ml of Iscove's enriched DMEM. The cells were washed with Iscove's DMEM with 10% foetal calf serum, and the assay run in Iscove's DMEM without foetal calf serum at $37^o$. Freshly cloned cells produced an initial cell culture supernatant which would also inhibit the mouse DNP plaque assay, as well as 5'-Nucleotidase, but this ability was soon lost. It was found that Clones 99 and 57 could be stimulated to produce more factor with a dilution of 1:4 of a monoclonal IgM cell culture supernatant against an irrelevant antigen, and their ability to inhibit plaque assays was restored. 99 cells also responded to an $IgG_1$ cell culture supernatant. None of the monoclonal antibody supernatants on their own were found to be inhibitory, either as regards 5'-nucleotidase or the plaque assay.

The results are shown in the table.

Table showing inhibition of human tonsil 5'-nucleotidase and mouse primary immunoglobulin response by cell culture supernatants.

| Clone and Supernatant | Tonsil 5'-N | Plaques |
|---|---|---|
| Sample volume | 1 ml | 0.5 ml |
| 57 + IgM | 0.84 | 0.26 |
| 57 + $IgG_1$ | 0.97 | 0.67 (?) |
| 99 + IgM | 0.62 | 0.27 |
| 99 + $IgG_1$ | 0.77 | 0.30 |
| IgM | 1.02 | 1.06 |
| $IgG_1$ | 1.10 | 1.13 |
| 92* | 0.77 | 0.38 |
| 99* | 0.79 | 0.44 |

Monoclonal antibodies were produced in DMEM. The cell culture supernatants were diluted 1:4 with Iscove's enriched DMEM, and added to clones of 57 and 99 which were apparently not producing factor, having had several changes of medium since an initially positive 5'-nucleotidase result on cloning. 92* and 99* were the initially positive results from clones grown in RPMI 1640 in 3.5 cm diameter Costar wells. All cell culture media contained antibiotics and 10% inactivated foetal calf serum.

To measure IgM plaques the goat anti DNP serum was ommitted from the assay. Two experiments indicated that ascitic fluid from clones 92 and 99 were not inhibitory.

0.2 ml ascitic fluid was used, with 0.2 ml inactivated foetal calf serum as control. The number of plaques obtained were only about 1/10 of those found when the anti DNP Ig was present.

| Clone | No. IgM plaques | Control Plaques |
|-------|-----------------|-----------------|
| 92 | 6,11 | 13,14 |
| 92 | 24 | 24 |
| 99 | 24 | 24 |

The ascitic fluid from mice injected with monoclonal antibodies showed some ability to inhibit 5'-Nucleotidase on tonsils and the mouse IgG plaque assay, as did apparently negative clones of 99. It is thought that the mouse reacted against the monoclonal antibodies and produced the factor in an attempt to switch off antibody production by the cells, and that the apparently negative 99 clone was a weak producer of factor. The non producing cell lines X-63 and SP2/0 can be grown in the ascitic cavities of mice, as can NS1, a producer of lambda light chains. However they provoke some immune reaction in the mouse. $2 \times 10^7$ cells were injected, and the ascitic fluid harvested 4 to 6 weeks later. As can be seen from the table, they failed to inhibit tonsil 5'-nucleotidase, but did inhibit the mouse IgG plaque assay. Ten times as many cells were injected as were required for tumours of the factor-producing or ordinary hybridoma cells, and the tumours take longer to grow.

Table showing inhibition by other mouse ascitic fluids Volume 0.2 ml

| Clone | Tonsil 5'-N | Mouse IgG Plaques |
|---|---|---|
| 99 -ve(?) | 0.90 | 0.90 |
| 99 -ve(?) | 0.87 | 0.74 |
| Monoclonal IgG | 0.83 | 0.43 |
| Monoclonal IgM | 0.82 | 0.75 |
| SP2/0 | 0.98 | 0.08 |
| X-63 | 0.95 | 0.02 |
| NS1 | 0.97 | 0.10 |

The inhibition of the plaque assay by the monoclonal antibodies is much less than that by the positive factor producing clones, see figure (1).

Inhibition of Human IgG Plaques

The tonsil cells used for the 5'-Nucleotidase assays were thawed and cultured for 5 days in Iscove's enriched DMEM with 10% foetal calf serum, antibiotics and fungizone (amphotericin B) at the concentration recommended by the manufacturer. One microgram $ml^{-1}$ pokeweed mitogen was included in the medium, and the cell concentration was $2-5 \times 10^{6}$/ml. Sheep red blood cells were labelled with Protein A using chromic chloride, (Hudson and Hay, loc. cit.).

The washed tonsil cells after stimulation with pokeweed mitogen were incubated with 0.2 ml portions of ascitic fluid or inactivated foetal calf serum (the control) in 1 ml Iscove's enriched DMEM for 1.5 hr at $37^{o}$.     The

cells were washed once in the Iscove's DMEM with foetal calf serum, then mixed with the protein A labelled sheep red cells, 1/50 dilution rabbit anti human immunoglobulin serum, and guinea pig complement in enriched DMEM with antibiotics. The plaques were left to develop overnight at $37^{\circ}$. The results of experiments with three tonsils are shown below.

Experiment 1

| Ascitic Fluid | Sample/Control |
|---|---|
| 92 | 0.23 |
| 99 | 1.90 |
| NS1 | 2.0 |

Experiment 2 and 3

| NS1 | 1.24, 1.24 |
|---|---|
| X-63 | 1.47, 1.24 |
| 57 | 1.26, 1.46 |
| 99 | 1.14, 1.28 |
| 92 | 0.63, 0.63 |

Cell counts showed that none of the ascitic fluids were cytotoxic for the tonsil cells, so the factor from 92 only produces an inhibition of human IgG production, of the order of 50%.

This result shows that the factors produced by the three clones are not identical at a molecular level, although they have similar molecular weights and apparently similar abilities to inhibit 5'-Nucleotidase.

Rowe and Johnson have shown that inhibition of

5'-nucleotidase by the competitive inhibitor, β-methylene adenosine diphosphate does not affect the production of pokeweed mitogen stimulated human lymphocytes in vitro, Biochem. Soc. Trans. (1979) $\underline{7}$, 997-998. Since the factor of the invention can be adsorbed by lymphocyte 5'-nucleotidase it should be possible to purify it by an affinity method.

Cell line 92 has been deposited at the Collection Nationale de Cultures de Micro-organisms, Institut Pasteur, Paris, France under the accession number I-280 on 2 March 1984.

CLAIMS:

1. A monoclonal factor that has an inhibitory effect on lymphocyte plasma membrane 5'-nucleotidase, has a negative effect on immunoglobulin production, and has a molecular weight of 44,000 daltons $\pm$ 7,000, as determined by gel filtration and/or polyacrylamide gel electrophoresis.

2. A monoclonal lymphocyte cell line that is capable of producing a factor as claimed in claim 1.

3. A cell line as claimed in claim 2, which consists of lymphocytes that have been hybridised with a cell line suitable for hybridisation, for example, hybrid lymphocyte/myeloma cells, hybrid lymphocyte/plasma cytoma cells, hybrid lymphocyte/hybridoma cells; or transformed lymphocyte cells.

4. A cell line as claimed in claim 2, having the characteristics of cell line 57, cell line 92, or cell line 99 as described herein.

5. A method of producing a cell line as claimed in claim 2, which comprises

(a) (i) fusing mammalian lymphocytes with a cell line suitable for hybridisation, for example, myeloma, plasma cytoma or hybridoma cells, or

(ii) transforming mammalian lymphocytes and then, after (i) or (ii),

(iii) cloning resulting hydridoma or transformed cells and selecting those clones that produce an inhibitor of lymphocyte plasma membrane 5'-nucleotidase and that have a negative effect on immunoglobulin production; or (b) cloning lymphocyte cells and selecting those that produce an inhibitor of lymphocyte plasma membrane 5'-nucleotidase and that have a negative effect on immunoglobulin production.

6.    A method as claimed in claim 5, wherein the lymphocytes are obtained from an animal that has been immunised previously.

7.    A method as claimed in claim 6, wherein the animal has been immunised previously with a cell line that is immunologically incompatible with the host animal and that is capable of producing antibodies.

8.    A method as claimed in claim 7, wherein the animal is non-human and the cell line is human myeloma cell line U 266 B1.

9.    A method as claimed in claim 5, wherein the lymphocytes are obtained from an animal that has been immunologically primed with an immunologically compatible antibody or with a cell line that is immunologically compatible and that is capable of producing immunologically compatible antibodies, or wherein the lymphocytes are primed in vitro with an immunologically compatible antibody.

10.    A method as claimed in any one of claims 5 to 9, wherein the myeloma cells used for fusion are SP2/O Ag 14 cells.

11.  A method as claimed in claim 5, wherein lymphocytes are transformed using the Epstein Barr virus.

12.  A method as claimed in any one of claims 5 to 11, wherein in the cloning procedure, after the second sub-clone, the hybrid or transformed cells are injected into the ascitic cavity of another animal immunologically compatible with the host animal.

13.  A method as claimed in claim 5, wherein Balb/c mice are immunised with human myeloma U 266 B1 living cells, and the resulting spleen B-lymphocytes are fused with SP2/O Ag 14 cells.

14.  A method as claimed in any one of claims 5 to 14, wherein additional antibody is present during the cloning procedure.

15.  A monoclonal lymphocyte cell line as claimed in claim 2, whenever produced by a process as claimed in any one of claims 5 to 14.

16.  A method of producing a factor as claimed in claim 1, which comprises (a) culturing a lymphocyte cell line as claimed in any one of claims 2 to 4 or claim 17 in the ascitic cavity of a host animal, and isolating the factor from the resulting ascitic fluid; or

(b) culturing a lymphocyte cell line as claimed in any one of claims 2 to 4 or claim 15 in cell culture, and isolating the factor from the resulting cell culture supernatant.

17. A method as claimed in claim 16, wherein additional antibody is present in the cell culture medium.

18. A factor as claimed in claim 1, whenever produced by a process as claimed in any one of claims 18 to 21.

19. A factor as claimed in claim 1, substantially as described herein with reference to Figures 1 to 4.

20. A pharmaceutical preparation which comprises a factor as claimed in claim 1, claim 18 or claim 19, in admixture or conjunction with a pharmaceutically suitable carrier.

21. A factor as claimed in claim 1, claim 18 or claim 19, for use as a therapeutic agent.

22. A monoclonal factor that has an inhibitory effect on lymphocyte plasma membrane 5'-nucleotidase, has a negative effect on immunoglobulin production, and has a molecular weight of $60,000 \pm 5,000$ when determined by polyacrylamide gradient gel electrophoresis of the unreduced molecule.

0118311

1/5

■ 5'-NUCLEOTIDASE,
CLONES 57,92 AND 99

□ 5'-NUCLEOTIDASE,
MONOCLONAL
ANTIBODIES

● MOUSE DNP PLAQUE
ASSAY, CLONES 92
AND 99

○ MOUSE DNP PLAQUE
ASSAY, MONOCLONAL
ANTIBODIES

VOLUME OF ASCITES FLUID, ML.

RATIO SAMPLE/CONTROL

*Fig.I.*

|   | M | Ig M |
|---|---|------|
|   | G | Ig G |
|   | A | ALBUMIN |
|   | F | FACTOR OF THE INVENTION |
|   | L | LIGHT CHAIN |
|   | P | PEPTIDE BAND |

FIG.2.

0118311

FIG.3.

0118311

THE MOLECULAR WEIGHT SCALE IS IN KILODALTONS, KD

F IS THE FACTOR OF THE INVENTION

FIG.4.

FIG. 5.

A   ALBUMIN

F   FACTOR OF THE INVENTION

*   RADIOACTIVE, TRACKS 1,3,6,8.